# EUROPÄISCHE PATENTSCHRIFT

(11) **EP 1 446 175 B1**
(45) Veröffentlichungstag und Bekanntmachung des Hinweises auf die Patenterteilung: **08.11.2006**
(21) Anmeldenummer: 02771970.7
(22) Anmeldetag: 05.11.2002
(51) Int. Cl.: A61M 5/142, A61M 39/28

(54) **Infusionspumpe**
Infusion pump
Pompe à perfusion

(30) Priorität: 15.11.2001 CH 20972001; 15.03.2002 CH 4492002
(43) Veröffentlichungstag der Anmeldung: 18.08.2004
(73) Patentinhaber: ARCOMED AG, 8105 Regensdorf (CH)
(72) Erfinder: VOLLENWEIDER, Marc, CH-8106 Adlikon (CH)
(74) Vertreter: Liebetanz, Michael
(86) Internationale Anmeldenummer: PCT/CH2002/000594
(87) Internationale Veröffentlichungsnummer: WO 2003/041787

(56) Entgegenhaltungen:
- EP-A- 0 447 985
- EP-A- 0 718 006
- US-A- 4 278 085
- US-B1- 6 196 519

## Beschreibung

Die Erfindung betrifft eine Infusionspumpe, mit der über einen Infusionsschlauch Flüssigkeiten förderbar sind, mit den Merkmalen der Anspruchs 1.

Eine solche Infusionspumpe ist aus der US 4,278,085 bekannt, bei der ein Drucksensor auf ein abgeschlossenes Segment einwirken kann, um als Ziel dieser Messung das Aufrechterhalten eines gewissen Drucks zu erreichen.

Infusionen sind eine Routineversorgung bei ärztlichen Behandlungen. Sie laufen über längere Zeiträume ab. Einerseits besteht zum Durchführen einer solchen Infusion die rein mechanische Lösung, bei der die Infusionsflüssigkeit über die Schwerkraft in den Körper des Patienten geleitet wird. Hier bestehen mechanische Dosierungselemente, z.B. den Schlauch klemmende ventile, die direkt auf dem Schlauch angebracht sind. Da diese direkt auf den Schlauch einwirken, ist durch den behandelnden Arzt oder die Krankenschwester eine Kontrolle über die Durchflussmenge gegeben, wenn über dieses Element eingestellt wird.

Alternativ besteht auch die Möglichkeit, eine Infusion mit Hilfe von sogenannten Infusionspumpen zu steuern, die auch die Möglichkeit bieten, die Durchflussmenge zeitabhängig zu steuern und über Sensoren auch Warnsignale abgeben können.

Dabei wird das Verbrauchsteil Schlauch, mit dem Infusionsbeutel an dem einen Ende und eventuell mit dem besagten auf dem Schlauch aufgesetzten Dosierungselement in die Infusionspumpe eingelegt, woraufhin eine entsprechend in der Pumpe vorgesehene Mechanik den Schlauch manipuliert, um eine vorbestimmte Durchflussgeschwindigkeit einzustellen und zu erhalten. Da die Steuerung des Durchflusses nun diesem Gerät obliegt, ist das eventuell noch vorhandene Dosierungselement, welches eine manuelle Durchflusssperre bildet auf offen gestellt, so dass die Steuerung des Durchflusses ausschliesslich der Infusionspumpe unterliegt. Beim Öffnen des Gerätes und der Herausnahme des Schlauches ist allerdings darauf zu achten, dass das besagte Dosierungselement, die manuelle Durchflusssperre, aktiviert ist, da ansonsten unkontrolliert die Infusionsflüssigkeit unter dem gegebenenfalls hohen Druck direkt in den Patienten läuft.

Zur Lösung dieser Problematik bei Infusionspumpen ist ein scheibenförmiges Kunststoffteil bekannt, welches einen Längsschlitz aufweist. An dem einen Ende des Längsschlitzes ist eine zum Beispiel kreisrunde Öffnung vorgesehen, die einen grösseren Durchmesser als die schmale Breite des Längsschlitzes aufweist. Durch die runde Öffnung wird oder ist vorab der Schlauch gelegt. Beim Einlegen des Schlauches in die Infusionspumpe wird dieses scheibenförmige Kunststoffteil in eine entsprechende komplementäre Ausnehmung in der Infusionspumpe eingesetzt und zwangsweise arretiert. Bei Entnahme des Schlauches wird dieser ebenfalls zwangsweise mit einem ersten Kraftaufwand in das schmälere Langloch überführt. Erst wenn dies geschehen ist, kann mit einem gegenüber dem ersten Kraftaufwand grösseren Kraftaufwand das scheibenförmige Kunststoffteil aus der Halterung herausgenommen werden. Somit wird durch Quetschung des Schlauches ein Durchfluss der unter Schwerkraft anstehenden verbleibenden Infusionsflüssigkeit sicher unterbunden.

Diese Vorrichtungen aus dem Stand der Technik sind aufwendig einzusetzen und auch nicht 100% sicher.

Darüber hinaus vermeiden diese Elemente nicht das falsche Einsetzen des Schlauches in dem Sinne, dass die Pumpe Flüssigkeit entgegen der Infusionsrichtung befördert.

Schliesslich kann der sogenannte Free-Flow, das freie Fliessen eines Medikamentes in den Patienten selbst bei aufwendigen Vorrichtungen nach dem Stand der Technik dadurch passieren, dass die Türe an sich undicht ist, zum Beispiel weil die Kraft, um das Besteck abzuquetschen nicht oder nicht mehr genügend ist. Es kann die Mechanik altern oder verschmutzen mit der Folge, dass das Infusionsbesteck nicht korrekt abgequetscht wird. Neben dem erwähnten falschen Einlegen des Infusionsbesteckes kann auch ein falsches Infusionsbesteck als solches eingelegt worden sein, zum Beispiel ein zu hartes oder ein zu dünnes, so dass die sich schliessende Tür den Schlauch nicht korrekt verschliessen kann.

Solche Probleme können auf Grund der bestehenden Toleranzen auch mit Tropfendetektoren nicht immer sicher erkannt werden; gleiches gilt natürlich auch für Durchflussensoren, die bereits zumeist ungenauer in der Auslegung sind.

Um diese Probleme zumindest ansatzweise anzugehen, werden typischerweise die Druckverhältnisses der Pumpentüre von solchen Infusionsgeräten regelmässig, zum Beispiel alle 24 Monate kontrolliert. Damit besteht natürlich eine mit der Ferne zum letzten Prüfungstermin wachsende Unsicherheit bezüglich der Funktionssicherheit der Geräte.

Ausgehend vom diesem Stand der Technik liegt der Erfindung die Aufgabe zugrunde, eine verbesserte Infusionspumpe mit einer Sicherheitsvorrichtung anzugeben.

Diese Aufgabe wird erfindungsgemäss mit den Merkmalen des Anspruchs 1 gelöst.

Mit einer solchen Ausgestaltung der Infusionspumpe ist es möglich, bei in dem abgeschlossenen Segment zwischen der Pumpmechanik und dem den Infusionsschlauch abschliessenden Element gesteuert einen Druck aufzubauen, dessen Grösse und Entwicklung in der Steuervorrichtung zu überwachen und bei Fehlfunktionen, die sich beispielsweise aus einer Abweichung von Kalibrierungskurven ergeben können, die Funktion der Infusionspumpe zu untersagen und eine entsprechende Ausgangsmeldung zu erzeugen.

Dadurch, dass, das quetschende und als Klemmventil ausgebildete Element in eine Halterung in der Infusionspumpe einsetzbar ist, kann das Klemmventil auf dem Infusionsschlauch vorgesehen sein. Das Klemmventil, das eine rastbare geschlossene Stellung aufweisen kann, ist in die Halterung in der Infusionspumpe formschlüssig einsetzbar, wobei in der Infusionspumpe ein Antriebselement vorgesehen ist, mit dem das Klemmventil aus der offenen Stellung in die rastbare geschlossene Stellung und vice versa überführbar ist.

Durch die Zwangsauslösung der rastbaren Stellung bei Öffnen der Infusionspumpe, entweder elektrisch oder mechanisch, kann sichergestellt werden, dass unabhängig von der Stellung des zumeist noch vorhandenen manuellen Dosierungselement der Zufluss von Infusionsflüssigkeit mit Öffnen der Infusionspumpe und damit bereits vor der Entnahme des Schlauches sicher unterbunden ist.

Die durch die Sicherungsvorrichtung gewährleistete zusätzliche Überprüfung des quetschenden Elementes auf Dichtheit geschieht vorteilhafterweise nach jedem Schliessen der Tür der Infusionspumpe.

Bei einer vorteilhaften Ausgestaltung ist das Ventil asymmetrisch ausgeformt, so dass gewährleistet wird, dass es immer in der richtigen Richtung in die Infusionspumpe eingelegt wird, so dass die Einbaurichtung des Schlauches immer richtig ist.

Vorteilhafterweise geschieht das Öffnen und Schliessen durch einen Exzenter, der mechanisch angetrieben ist, so dass die Funktion der Durchflusssperre in einfacher Weise geprüft werden kann.

Nachfolgend wird die Erfindung anhand eines bevorzugten Ausführungsbeispiels anhand der beigefügten Zeichnungen näher erläutert. Es zeigen:
- Fig. 1: eine schematische Vorderansicht einer offenen Infusionspumpe mit eingelegtem Schlauch,
- Fig. 2: eine perspektivische Darstellung eines Ausschnittes dieser Infusionspumpe mit einer Durchflusssperre im offenen Zustand,
- Fig. 3: eine Draufsicht auf die Durchflusssperre der Figur 2,
- Fig. 4: eine perspektivische Darstellung der Durchflusssperre nach Figur 2 im geschlossenen Zustand, und
- Fig. 5: eine Draufsicht auf die Durchflusssperre der Fig. 4.

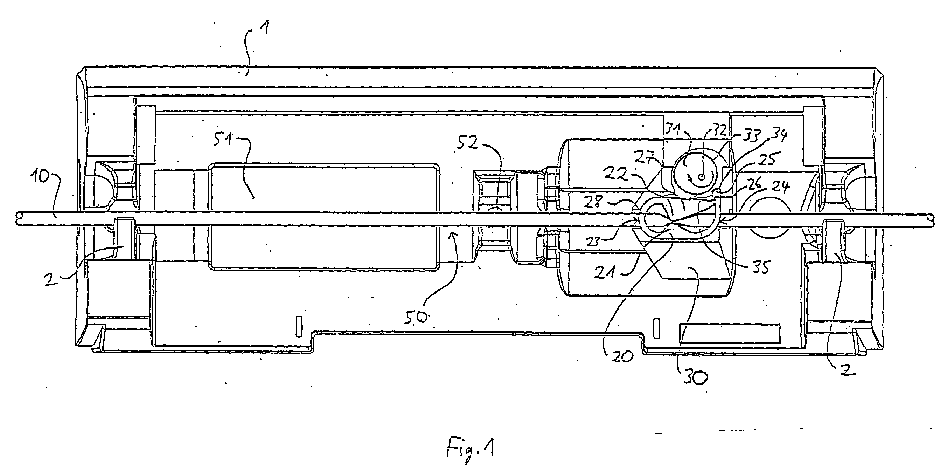

Die Figur 1 zeigt eine schematische Ansicht einer geöffneten Infusionspumpe 1, das heisst einer Infusionspumpe 1 mit geöffnetem Deckel. Diese verfügt zumeist auf ihren beiden Seiten über jeweils einen Haltehaken 2, in dem bei geöffnetem Deckel, wie es in der Fig. 1 dargestellt ist, ein Infusionsschlauch 10 einsetzbar ist. Auf dem Infusionsschlauch 10 ist eine Durchflusssperre 20 aufgesetzt, die ein quetschendes Element darstellt.

Die Durchflusssperre 20 ist in der Fig, 1 im geschlossenen Zustand dargestellt, was daran erkennbar ist, dass zwei gegenüberliegende Quetschwülste 21 und 22 den Schlauch 10 bis zum Verschluss zusammendrücken. Wie noch in den weiteren Figuren näher zu erkennen sein wird, verfügt die Durchflusssperre 20 an ihren beiden Enden über eine Bohrung 23 bzw. 24, durch die der unverformte Schlauch 10 durchgeführt ist. Das eine Ende 25 der Durchflusssperre verfügt über einen Rasthaken 26, hinter dem das andere Ende 27 der Durchflusssperre 20 einrastbar ist. Dieses Ende 27 ist durch den gebogenen Teilabschnitt 28 mit dem Durchbruch 23 flexibel und kann bis zum Zusammenquetschen des Schlauches 10 über den an ihm bestehenden Wulst 22 gedrückt werden.

Die Durchflusssperre liegt in einer in der Infusionspumpe 1 vorgesehenen Durchflusssperrenhalterung 30 in einem Formschluss ein. Da die Durchflusssperrenhalterung 30 entsprechend der Aussenform der nicht komprimierten (= offenen) Durchflusssperre 20 ausgeformt ist, ist die Durchflusssperre 20 nur in der in der Figur 1 dargestellten Richtung einsetzbar, so dass sichergestellt ist, dass die Einbaurichtung richtig ist. In der um 180 Grad verdrehten Richtung würde das freie Ende 25 nicht in dem für den abgerundeten Abschnitt 28 vorgesehenen Teil einlegbar sein.

Die Durchflusssperrenhalterung 30 verfügt neben den Durchbrüchen für das Einlegen des Schlauches 10 über eine weitere seitliche Öffnung, in der ein zylinderförmiges Element 31 in exzentrischer Lage um eine Achse 32 rotiert. Dabei drückt der Zylindermantel 33 der exzentrischen Scheibe 31 einerseits auf eine Endfläche 34 des Endes 25 der Durchflusssperre 20, um den Eingriff des Rasthakens 26 zu lösen und das Ventil zu öffnen. Bei einer weitergehenden Drehung der Scheibe 31 in Richtung des Uhrzeigersinnes drückt der Mantel 33 dann mehr und mehr auf die Rückfläche 35 des anderen freien Endes und drückt dieses nieder, so dass die Wülste 21 und 22 den Schlauch 10 quetschen und der Rasthaken 26 schlussendlich in das Ende 25 einrastet.

Funktionselemente der Infusionspumpe 1, die den Durchfluss an sich steuern sind nicht Gegenstand der Erfindung. Wesentlich ist aber die Funktion der Infusionspumpe 1 bei einem Öffnen des Dekkels der Infusionspumpe 1. Dabei ist der Deckel insofern mit der Steuerung der Exzenterscheibe 31 verbunden, als dass diese Scheibe 31 bei Öffnen des Deckels der Infusionspumpe 1 automatisch in die in der Fig. 1 dargestellte verschlossene Raststellung übergeht.

Bei einem anderen Ausführungsbeispiel ist es auch möglich, dass anstelle der Scheibe 31 ein Hebel vorgesehen ist, der das freie Ende 27 der Durchflusssperre 20 niederdrückt, und bei anderen Ausgestaltungen kann an Stelle eines elektrischen Antriebes zum Verriegeln des Ventils bei Öffnen des Deckels auch eine federbetätigte Vorrichtung vorgesehen sein, die bei Öffnen des Deckels der Infusionspumpe 1 auslöst. Sie kann durch das Schliessen des Deckels vorgespannt werden.

Ferner kann ein Kontaktschalter vorgesehen sein, mit dem bei einem Öffnen des Deckels der Infusionspumpe 1 diese Information an eine Sicherheitsvorrichtung übermittelt wird, die im Gerät der Fig. 1 nicht dargestellt ist, da sie zusammen mit der weiteren Elektronik im Innern des Gerätes vorgesehen ist. Mit dem Bezugszeichen 51 ist die Pumpenmechanik bezeichnet, die den Schlauch 10 umgibt. Damit wird ein Bereich 50.zwischen dieser Pumpenmechanik 51 und der Durchflusssperre 20 definiert. In diesem Bereich ist auf dem Schlauch 10 ein Drucksensor 52 vorgesehen, mit dem der Fluiddruck in dem besagten Teilbereich 50 feststellbar ist.

Die Funktion der Sicherungsvorrichtung ist wie folgt. Wie in den nachfolgenden Beschreibungen erläutert, kann die Durchflusssperre 20 auf "geschlossen" eingestellt werden. Dann wird von der Sicherungsvorrichtung die Pumpmechanik 51 gestartet, was sofort zu einem Druckanstieg in dem Bereich 50 führt, ohne dass Flüssigkeit oder Druck in den Patienten gelangt. Der Druckanstieg wird von dem Drucksensor 52 registriert, der die entsprechenden Signale an die Steuervorrichtung übermittelt.

Somit ist es zuallererst direkt möglich, automatisch festzustellen, ob sowohl Pumpmechanik 51 als auch Durchflusssperre 20 dicht sind. Sollte dies nicht der Fall sein, was zum Beispiel durch eine schleichende Undichtheit des Besteckes möglich sein kann, so wird die Pumpmechanik 51 sofort gestoppt und/oder rückwärts laufend betätigt, um weder Druckanstieg noch einen Flüssigkeitsfluss in Richtung Patienten zu erhalten.

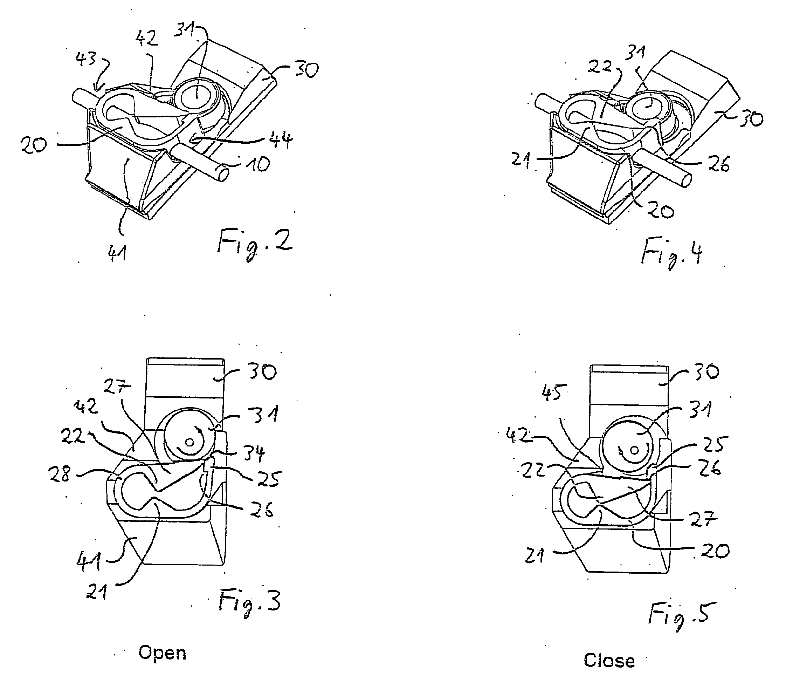

Bei einem bestimmten erreichten oder zurückgenommenen Druck in dem Bereich 50 kann die Durchflusssperre 20 geöffnet werden, um deren Funktion zu überprüfen, indem der nun in dem Bereich 50 entstehende Druckunterschied durch denselben Drucksensor 52 aufgenommen und die Steuereinrichtung weiterverarbeitet wird. Somit kann festgestellt werden, ob die Duichflusssperre 20 richtig eingelegt wurde beziehungsweise richtig funktioniert. Es könnte dann auch festgestellt werden, ob eine äussere Klemme geschlossen wurde, was mit dem Druckanstiegstest eventuell nicht zweifelsfrei ermittelbar war, oder dass anstelle der Durchflusssperre 20 gemäss der hier zu den Figuren 2 bis 5 beschriebenen Art nur eine normale Rollenklemme geschlossen wurde.

In den weiteren Figuren wird in anderen Darstellungen die Wirkung der Durchflusssperre 20 näher erläutert.

Die Fig. 2 zeigt in einer schematischen perspektivischen Ansicht die Durchflusssperrenhalterung 30 mit eingelegtem Schlauch 10, auf dem eine Durchflusssperre 20 angeordnet ist. Gleiche Merkmale tragen in allen Figuren dieselben Bezugszeichen. Dabei zeigen Fig. 2 und 3 den offenen Zustand der Durchflusssperre 20, bei dem die Steuerung der Infusionspumpe 1 den Durchfluss regelt.

Insbesondere sind in der Fig. 2 die beiden gegenüberliegenden Seitenwände 41 und 42 der Durchflusssperrenhalterung 30 zu erkennen, die einen grossen Teil der Durchflusssperre 20 formschlüssig umfassen. Mit den Bezugszeichen 43 und 44 sind die seitlichen Öffnungen bezeichnet, durch die der Schlauch durch die Durchflusssperrenhalterung 30 hindurchgeführt wird.

Schliesslich zeigt die Fig. 2 anschaulich, dass zur Sicherung der Funktion der Durchflusssperre das Vorsehen einer Durchflusssperrenhalterung 30 in einer handelsüblichen Infusionspumpe ausreichend ist, sofern die Exzenterscheibe 31 oder ein vergleichbares vorgesehenes Element durch das Öffnen des Deckels und/oder durch Knopfdruck zur Funktionsüberprüfung aktivierbar ist.

Die Fig. 3 zeigt den in der Fig. 2 dargestellten Gegenstand in einer schematischen Draufsicht ohne Schlauch 10. Durch eine Drehung der Scheibe 31 entgegen dem Uhrzeigersinn ist die Fläche 34 des freien Endes 25 seitlich aufgebogen worden, um das Ventil zu öffnen. Das freie Ende 27 greift nicht in den Rasthaken 26 ein.

Die Fig. 4 zeigt in einer schematischen perspektivischen Ansicht die Durchflusssperrenhalterung 30 mit eingelegtem Schlauch 10, auf dem die Durchflusssperre 20 angeordnet ist. Fig. 4 und 5 zeigen den geschlossenen Zustand der Durchflusssperre 20, bei dem der Schlauch aus der Infusionspumpe 1 entnommen werden kann.

Insbesondere ist in der Fig. 4 zu erkennen, das die beiden Wülste 21 und 22 den Schlauch quetschen. Die Wülste sind in der Draufsicht dreieckig mit einer geraden Abschlusskante, um jeweils eine tangential zum Schlauch 10 verlaufende Quetschlinie zu definieren.

Schliesslich zeigt die Fig. 5, dass bei der Sicherung der Funktion der Durchflusssperre zwischen der Fläche 42 und dem freien Ende 27 ein Zwischenraum 45 entsteht, wenn durch die Exzenterscheibe 31 oder ein vergleichbares Element vor oder beim Öffnen des Deckels das freie Ende 27 in den Rasthaken 26 des anderen Endes 25 der Durchflusssperre 20 rastet.

Aufgrund der Einfachheit der einstückigen Durchflusssperre 20 kann diese serienmässig auf Schläuchen 10 vorgesehen sein, unabhängig davon, ob eine Infusion mit einem manuell einzustellenden Dosierelement oder mit einer Infusionspumpe 1 verabreicht werden soll. Die Durchflusssperre 20 stört ausserhalb einer Infusionspumpe 1 nicht die Funktion eines Dosierelementes, da sie nur die Stellungen offen und geschlossen kennt und somit ein irrtümlicher Verschluss des Schlauches sofort erkannt wird.

Mit einer Vorrichtung und einem Verfahren mit den Merkmalen der beiliegenden Ansprüche kann die Sicherheit der Klemme automatisch überprüft werden, indem bei jedem Schliessen der Tür der Vorrichtung die Steuervorrichtung das oben genannte Verfahren ablaufen lässt. Dabei muss die Klemme nicht unbedingt eine am Infusionsbesteck montierte Klemme sein, sondern kann auch eine Abklemmvorrichtung der Pumpe sein, die ein normales Besteck öffnen und schliessen kann.

Da das Klemmventil 20 asymmetrisch ausgeformt ist, so dass es in Bezug auf die Ausrichtung des Infusionsschlauches 10 in longitudinaler Richtung nur eineindeutig in die komplementäre Halterung 30 einsetzbar ist, kann das Einhalten der richtigen Einbaurichtung des Infusionsschlauches 10 in der Infusionspumpe 1 gewährleistet werden.

Mit der erfindungsgemässen Vorrichtung können auch Okklusionen stromab oder stromauf des Schlauchsegmentes 50 automatisch festgestellt und über Anzeigen bei der Infusionspumpe gemeldet werde.

Wesentlich ist die durch die Erfindung gegebene Möglichkeit, dass durch Einlegen eines Infusionsschlauches in die Infusionspumpe ein Automatismus getriggert wird und ablaufen kann, mit dem wesentliche Sicherheitsgrundfunktionen von Schlauch (insbesondere innerhalb der Infusionspumpe aber auch ausserhalb der Infusionspumpe) und Pumpe (Schlauch und Klemmentyp kompatibel mit dieser Art von Infusionspumpe, richtig eingelegt und richtig orientiert eingelegt) geprüft werden können.

## Patentansprüche

1. Infusionspumpe (1), in die ein Infusionsschlauch (10) einsetzbar (2) ist, mit einer Pumpmechanik (51) zum Einwirken auf den besagten Infusionsschlauch (10), so dass durch den Infusionsschlauch (10) Flüssigkeiten förderbar sind, mit einem stromabwärts der Pumpmechanik angeordneten Drucksensor (52) zum Aufnehmen von Druckvariationen im Infusionsschlauch und mit einer an den Ausgang des Drucksensors angeschlossenen Steuervorrichtung für die Pumpmechanik und für ein den Infusionsschlauch (10) quetschendes Element (20; 21, 22), **dadurch gekennzeichnet, dass** die Infusionspumpe (1) stromabwärts des Drucksensors über eine Halterung (30) verfügt, in welche ein den Infusionsschlauch (10) quetschendes und als Klemmventil ausgebildetes Element (20; 21, 22) einsetzbar ist, und dass die Steuervorrichtung derart ausgebildet ist, dass mit ihr ein in die Halterung eingesetztes Klemmventil (20; 21, 22) in eine geschlossene Stellung überführbar und die Pumpmechanik (51) zur Veränderung der Druckverhältnisse in einem durch die Pumpmechanik und das Klemmventil abschliessbaren Schlauchsegment (50) ansteuerbar ist, so dass mit den über den Drucksensor gemessenen Werten Funktionen der Infusionspumpe (1) und des Infusionsschlauches (10) überprüfbar sind.

2. Infusionspumpe nach Anspruch 1, **dadurch gekennzeichnet, dass** die Steuervorrichtung derart ausgebildet ist, dass mit ihr ein in die Halterung eingesetztes Klemmventil (20; 21, 22) in eine geöffnete Stellung überführbar und die Pumpmechanik (51) zur Veränderung der Druckverhältnisse in einem durch die Pumpmechanik und das Klemmventil dann nicht mehr abgeschlossenen Schlauchsegment (50) ansteuerbar ist, so dass mit den über den Drucksensor (52) oder einen weiteren Sensor gemessenen Werten Funktionen der Infusionspumpe (1) und des Infusionsschlauches (10) überprüfbar sind.

3. Infusionspumpe nach Anspruch 1 oder 2, **dadurch gekennzeichnet, dass** als Funktion der Infusionspumpe (1) die Dichtheit des Infusionsschlauches (10) in der Pumpmechanik überprüfbar ist.

4. Infusionspumpe nach Anspruch 1, 2 oder 3, **dadurch gekennzeichnet, dass** mit der Halterung (30) das korrekte Einlegen des Infusionsschlauches (10) und des quetschenden Elementes (20; 21, 22) überprüfbar ist.

5. Infusionspumpe nach einem der Ansprüche 1 bis 4, weiterhin umfassend ein für den Infusionsschlauch (10) vorgesehenes Klemmventil (20, 21, 22) **dadurch gekennzeichnet, dass** das Klemmventil (20, 21, 22) asymmetrisch ausgeformt ist und die Halterung (30) eine richtungsbezogene Halterung ist, die komplementär zu dem asymmetrisch ausgebildeten Klemmventil ausgebildet ist, so dass durch die Infusionspumpe (1) die korrekte Orientierung des Infusionsschlauches (10) und des Klemmventils (20; 21, 22) überprüfbar ist.

6. Infusionspumpe nach Anspruch 5, **dadurch gekennzeichnet, dass** das Klemmventil (20) eine rastbare geschlossene Stellung aufweist und es in die Halterung in der Infusionspumpe (1) formschlüssig (30) einsetzbar ist, und dass in der Infusionspumpe (1) ein Antriebselement (31) vorgesehen ist, mit dem das Klemmventil (20) aus der offenen Stellung in die rastbare geschlossene Stellung und vice versa überführbar ist.

7. Verfahren zum Betreiben einer Infusionspumpe nach einem der Ansprüche 1 bis 6, **dadurch gekennzeichnet, dass** die Steuervorrichtung der Infusionspumpe ein Startsignal erhält, welches durch das Schliessen der Tür der Infusionspumpe oder einen manuellen externen Startschalter erzeugt wird, dass die Steuervorrichtung die Pumpmechanik (51) der Infusionspumpe mit einem Steuersignal beaufschlagt, so dass der Druck in dem Bereich zwischen Pumpmechanik und Klemmventil (20) in einem vorbestimmten Zeitablauf erhöht und anschliessend gehalten oder erniedrigt wird, wobei zeitgleich von dem Drucksensor (52) für den in dem Infusionsschlauch (10) herrschenden Druck repräsentative Daten an die Steuervorrichtung übermittelt werden, mit der durch einen Datenvergleich ein Ergebnissignal, welches für die Funktionsfähigkeit oder die Funktionsunfähigkeit der Infusionspumpe repräsentativ ist, erzeugbar ist.

8. Verfahren nach Anspruch 7, **dadurch gekennzeichnet, dass** die Steuervorrichtung ein Öffnen-Signal erzeugt und an das Klemmventil (20) übermittelt, auf welches hin das Klemmventil (20) geöffnet wird, und dass von dem Drucksensor (52) nach dem Öffnen des Klemmventils (20) der Druckabfall in dem Bereich zwischen Pumpmechanik und Klemmventil (20) erfasst und an die Steuervorrichtung übermittelt wird, in der durch einen Datenvergleich ein Ergebnissignal, welches für die Funktionsfähigkeit oder die Funktionsunfähigkeit des Öffnens des Klemmventils (20) repräsentativ ist, erzeugbar ist..

## Claims

1. An infusion pump (1), into which an infusion tube (10) is insertable, comprising a pump mechanism (51) to act upon said infusion tube (10) to deliver liquids via the infusion tube (10), a pressure sensor (52) arranged downstream of the pump mechanism to detect pressure variations within the infusion tube and a control device for the pump mechanism and for an element (20; 21, 22) squeezing the infusion tube (10), connected to the output of the pressure sensor, **characterized in that** downstream of the pressure sensor the infusion pump (1) comprises a holder (30), into which an element (20; 21, 22) squeezing the infusion tube (10) and forming a clamp valve is insertable, and **in that** the control device is arranged to move a clamp valve (20; 21, 22) positioned within the holder into a closed position and to control the pump mechanism (51) for a variation of the pressure conditions in a segment (50) which can be closed-off through the pump mechanism and the clamp valve, so that functions of the infusion pump (1) and of the infusion tube (10) can be checked with the measured values from the pressure sensor.

2. The infusion pump as claimed in claim 1, **characterized in that** the control device is arranged to move the clamp valve (20; 21, 22) inserted in the holder into an open position and to control the pump mechanism (51) for a variation of the pressure conditions in the segment (50), which is then no longer closed-off through the pump mechanism and the clamp valve, so that values measured with the aid of the sensor (52) or a further sensor allow to check functions of the infusion pump (1) and of the infusion tube (10).

3. The infusion pump as claimed in claim 1 or 2, **characterized in that** the tightness of the infusion tube (10) in the pump mechanism can be checked as the function of the infusion pump (1).

4. The infusion pump as claimed in claim 1, 2 or 3, **characterized in that** the holder (30) allow to check the correct insertion of the infusion tube (10) and of the squeezing element (20; 21, 22).

5. The infusion pump as claimed in one of preceding claims 1 to 4, further comprising a clamp valve (20; 21, 22) to be used with the infusion tube (10), **characterized in that** the clamp valve (20; 21, 22) is shaped asymmetrical and **in that** the holder (30) is a directional holder, being formed as complementary to the asymmetrical shaped clamp valve, so that the infusion pump (1) can check the correct orientation of the infusion tube (10) and of the clamp valve (20; 21, 22).

6. The infusion pump as claimed in claim 5, **characterized in that** the clamp valve (20) has a lockable closed position and can be inserted with a positive fit (30) into the holder in the infusion pump (1), and **in that** a drive element (31) is provided in the infusion pump (1), with which drive element (31) the clamp valve (20) can be moved from the open position into the lockable closed position and vice versa.

7. A method for operating the infusion pump as claimed in one of claims 1 through 6, **characterized in that** the control device obtains a start signal generated by the closing of the door of the infusion pump or by a manual external starter switch, **in that** the control device acts on the pump mechanism (51) of the infusion pump with a control signal so that the pressure in the area between pump mechanism and clamp valve (20) rises in a predetermined time period and is then maintained or lowered, while at the same time data representative of the pressure prevailing in the infusion tube (10) are transmitted from the pressure sensor (52) to the control device with which, by means of data comparison, a result signal can be generated which is representative of the functional ability or functional inability of the infusion pump.

8. The method as claimed in claim 7, **characterized in that** the control device generates an open signal and transmits this to the clamp valve (20), in response to which signal the clamp valve (20) is opened, and **in that** the pressure drop in the area between pump mechanism and clamp valve (20) is detected by the pressure sensor (52) after opening of the clamp valve (20) and is transmitted to the control device in which, by means of data comparison, a result signal can be generated which is representative of the functional ability or functional inability of the opening of the clamp valve (20).

## Revendications

1. Pompe à perfusion (1), dans laquelle un tube de perfusion (10) peut être introduit, avec une mécanique de pompe (51) pour agir sur ledit tube de perfusion (10) pour délivrer du liquide par le tube de perfusion (10), avec un détecteur de pression (52), arrangé en aval du mécanisme de pompe, pour détecter des variations de pression à l'intérieur du tube de perfusion et avec un équipement de contrôle pour le mécanisme de pompe et pour un élément (20, 21, 22) contusionnant le tube de perfusion (10), relié à la sortie du détecteur de pression, **caractérisée en ce qu'**en aval du détecteur de pression la pompe à perfusion (1) comprend un crochet (30), dans lequel un élément (20, 21, 22) contusionnant le tube de perfusion (10) et formant une valve de serrage peut être introduit et **en ce que** l'équipement de contrôle est arrangé pour déplacer la valve de serrage (20, 21, 22) positionnée à l'intérieur du crochet dans une position fermée et pour contrôler le mécanisme de pompe (51) pour une variation de conditions de pression dans un segment (50), qui peut être fermé par le mécanisme de pompe et la valve de serrage, pour que les fonctions de la pompe à perfusion (1) et du tube de perfusion (10) peuvent être vérifiées par les valeurs mesurées du détecteur de pression.

2. Pompe à perfusion selon la revendication 1, **caractérisée en ce que** l'équipement de contrôle est arrangé pour déplacer la valve de serrage (20, 21, 22) introduit dans le crochet dans une position ouverte et pour contrôler le mécanisme de pompe (51) pour une variation de conditions de pression dans le segment (50), qui n'est plus fermé par le mécanisme de pompe et la valve de serrage, pour que les valeurs mesurées à l'aide du détecteur (52) ou un détecteur supplémentaire permet de vérifier les fonctions de la pompe à perfusion (1) et du tube de perfusion (10).

3. Pompe à perfusion selon la revendication 1 ou 2, **caractérisée en ce qu** l'étanchéité du tube de perfusion (10) dans le mécanisme de pompe peut être vérifiée en tant que fonction de la pompe à perfusion.

4. Pompe à perfusion selon la revendication 1, 2 ou 3, **caractérisée en ce que** le crochet (30) permet de vérifier l'introduction correcte du tube de perfusion (10) et de l'élément contusionnant (20, 21, 22).

5. Pompe à perfusion selon une quelconque des revendications 1 à 4, comprenant aussi une valve de serrage (20, 21, 22) à être utilisé avec le tube de perfusion (10), **caractérisé en ce que** la valve de serrage (20, 21, 22) est formée d'une manière asymétrique et **en ce que** le crochet (30) est un crochet directionnel, formé d'une manière complémentaire à la valve de serrage asymétrique, pour que la pompe à perfusion (1) puisse vérifier l'orientation correcte du tube de perfusion (10) et de la valve de serrage (20, 21, 22).

6. Pompe à perfusion selon la revendication 5, **caractérisé en ce que** la valve de serrage (20) comprend une position qui peut être verrouillée et qui peut être introduite d'une manière géométrique dans le crochet (30) dans la pompe à perfusion (1), et **en ce qu'**un élément d'entraînement (31) est prévu dans la pompe à perfusion (1), avec lequel la valve de serrage (20) peut être déplacée d'une position ouverte dans une position verrouillée et vice versa.

7. Procédé pour utiliser la pompe à perfusion selon l'une des revendications 1 à 6, **caractérisé en ce que** l'équipement de contrôle obtient un signal de départ généré par la fermeture de la porte de la pompe à perfusion ou par un interrupteur extérieur manuel de marche, **en ce que** l'équipement de contrôle agit sur le mécanisme de pompe (51) de la pompe à perfusion avec un signal de contrôle pour que la pression dans la zone entre le mécanisme de pompe et la valve de serrage (20) monte dans une durée prédéterminée et soit ensuite maintenue ou abaissée, où, pendant le même temps, des données représentatives pour la pression prédominante dans le tube de perfusion (10) sont transmises du détecteur de pression (52) à l'équipement de contrôle avec lequel, par moyen de comparaison des données, un signal résultant peut être généré qui représente la capacité ou incapacité fonctionnelle de la pompe à perfusion.

8. Procédé selon la revendication 7, **caractérisé en ce que** l'équipement de contrôle génère un signal d'ouverture et transmet celui-ci à la valve de serrage (20), où, en réponse à ce signal, la valve de serrage (20) est ouverte, et **en ce que** la chute de pression dans la zone entre le mécanisme de pompe et la valve de serrage (20) est détectée par le détecteur de pression (52) après ouverture de la valve de serrage (20) et est transmis à l'équipement de contrôle, dans lequel, par comparaison des données, un signal résultant peut être généré qui représente la capacité ou l'incapacité fonctionnelle de l'ouverture de la valve de serrage (20).
